# EUROPEAN PATENT SPECIFICATION

(11) **EP 3 152 575 B1**
(45) Date of publication and mention of the grant of the patent: **21.08.2019**
(21) Application number: 14735774.3
(22) Date of filing: 05.06.2014
(51) Int. Cl.: G01N 33/569

(54) **ASSAY FOR ORAL INFLAMMATION**
TEST FÜR MUNDENTZÜNDUNG
TEST DE DÉTECTION D'UNE INFLAMMATION DE LA CAVITÉ BUCCALE

(43) Date of publication of application: 12.04.2017
(73) Proprietor: Colgate-Palmolive Company, New York, NY 10022 (US)
(72) Inventor: SREENIVASAN, Prem, Westfield New Jersey 07090 (US)
(74) Representative: Wibbelmann, Jobst
(86) International application number: PCT/US2014/041158
(87) International publication number: WO 2015/187169

(56) References cited:
- Anita Moosani ET AL: "Evaluation of Oral Neutrophil Levels as a Quantitative Measure of Periodontal Inflammatory Load in Patients with Special Needs", , 1 November 2012 (2012-11-01), XP55435006, Retrieved from the Internet: URL:https://tspace.library.utoronto.ca/bit stream/1807/33456/1/Moosani_Anita_201211_M Sc_thesis.pdf
- H. JENTSCH ET AL: "Lactoferrin and other markers from gingival crevicular fluid and saliva before and after periodontal treatment", JOURNAL OF CLINICAL PERIODONTOLOGY, vol. 31, no. 7, 1 July 2004 (2004-07-01), pages 511-514, XP55434986, DK ISSN: 0303-6979, DOI: 10.1111/j.1600-051X.2004.00512.x
- David A. Scott ET AL: "Neutrophils in Periodontal Inflammation" In: "Periodontal Disease", 1 January 2011 (2011-01-01), KARGER, Basel, XP55435002, ISSN: 1662-3770 ISBN: 978-3-8055-9833-0 vol. 15, pages 56-83, DOI: 10.1159/000329672, * the whole document *
- BHADBHADE SMRUTI JAYPRAKASH ET AL: "Correlation between probing pocket depth and neutrophil counts in dental plaque, saliva, and gingival crevicular fluid.", QUINTESSENCE INTERNATIONAL (BERLIN, GERMANY : 1985) FEB 2012, vol. 43, no. 2, February 2012 (2012-02), pages 111-117, XP008170850, ISSN: 1936-7163
- E. ADONOGIANAKI ET AL: "Lactoferrin in the gingival crevice as a marker of polymorphonuclear leucocytes in periodontal diseases", JOURNAL OF CLINICAL PERIODONTOLOGY, vol. 20, no. 1, 1 January 1993 (1993-01-01), pages 26-31, XP055131636, ISSN: 0303-6979, DOI: 10.1111/j.1600-051X.1993.tb01755.x
- MARTINS C A P ET AL: "CORRELATION OF LACTOFERRIN WITH NEUTROPHILIC INFLAMMATION IN BODY FLUIDS", CLINICAL AND DIAGNOSTIC LABORATORY IMMUNOLOGY, AMERICAN SOCIETY FOR MICROBIOLOGY, US, vol. 2, no. 6, 1 November 1995 (1995-11-01), pages 763-765, XP000570955, ISSN: 1071-412X
- MC MURRAY ET AL: "The relationship between elastase and lactoferrin in healthy, gingivitis and periodontitis sites", ORAL DISEASES, vol. 1, no. 3, 1 September 1995 (1995-09-01), pages 106-109, XP055131719, ISSN: 1354-523X, DOI: 10.1111/j.1601-0825.1995.tb00172.x
- A ZIA ET AL: "Oral biomarkers in the diagnosis and progression of periodontal diseases", BIOLOGY AND MEDICINE, vol. 3, no. 2, 23 October 2011 (2011-10-23), pages 45-52, XP055131496,
- LAURA ESTELA ET AL: "Innovative study on lactoferrin in periodontal disease Papel de la lactoferrina en enfermedades periodontales", REVISTA ODONTOLÓGICA MEXICANA, vol. 15, no. 4, 1 January 2011 (2011-01-01), pages 231-237, XP055131479,
- SUOMALAINEN ET AL: "Peroxidases, lactoferrin and lysozyme in peripheral blood neutrophils, gingival crevicular fluid and whole saliva of patients with localized juvenile periodontitis.", ORAL DISEASES, vol. 2, no. 2, 1 June 1996 (1996-06-01), pages 129-134, XP055131458, ISSN: 1354-523X
- NAPOLEON WASZKIEWICZ ET AL: "Decrease in salivary lactoferrin output in chronically intoxicated alcohol-dependent patients", FOLIA HISTOCHEMICA ET CYTOBIOLOGICA, vol. 50, no. 2, 5 July 2012 (2012-07-05), pages 248-254, XP055131462, ISSN: 0239-8508, DOI: 10.5603/FHC.2012.0024
- Anita Moosani ET AL: "Evaluation of Oral Neutrophil Levels as a Quantitative Measure of Periodontal Inflammatory Load in Patients with Special Needs", , 1 November 2012 (2012-11-01), XP55435006, Retrieved from the Internet: URL:https://tspace.library.utoronto.ca/bit stream/1807/33456/1/Moosani_Anita_201211_M Sc_thesis.pdf
- H. JENTSCH ET AL: "Lactoferrin and other markers from gingival crevicular fluid and saliva before and after periodontal treatment", JOURNAL OF CLINICAL PERIODONTOLOGY, vol. 31, no. 7, 1 July 2004 (2004-07-01), pages 511-514, XP55434986, DK ISSN: 0303-6979, DOI: 10.1111/j.1600-051X.2004.00512.x
- David A. Scott ET AL: "Neutrophils in Periodontal Inflammation" In: "Periodontal Disease", 1 January 2011 (2011-01-01), KARGER, Basel, XP55435002, ISSN: 1662-3770 ISBN: 978-3-8055-9833-0 vol. 15, pages 56-83, DOI: 10.1159/000329672, * the whole document *
- J. S. Bender ET AL: "Novel rinse assay for the quantification of oral neutrophils and the monitoring of chronic periodontal disease", Journal of Periodontal Research, vol. 41, no. 3, 1 June 2006 (2006-06-01), pages 214-220, XP55480302, ISSN: 0022-3484, DOI: 10.1111/j.1600-0765.2005.00861.x

## Description

### BACKGROUND

Oral tissue inflammation can be caused by surgery, localized injury, trauma, necrosis, improper oral hygiene or various systemic origins. Gingiva (gums) are part of the soft tissue lining in the mouth surrounding the teeth. Gingivitis is an inflammation of the gums that often appears as swollen, red, or bleeding gums. Gingivitis is the inflammation or infection of the gums and the alveolar bones that support the teeth. Periodontitis is a progressively worsened state of disease as compared to gingivitis, where the gums are inflamed and begin to recede from the teeth and pockets form, which ultimately may result in destruction of the bone and periodontal ligament.

It is generally believed that plaque-forming bacteria that live in the mouth and on tooth surfaces are a cause of oral inflammation, such as gingivitis, and certain substances released by the bacteria cause the oral inflammation. Among other things, the bacteria associated with oral inflammation generate, as waste products, metabolites including gases such as hydrogen sulfide, methyl mercapatan and other sulfur gases. These gases and metabolites are believed to be one of the major contributing causes of the odor of halitosis.

Clinical observations from the 1960's have established the relationship between accumulated dental plaque and the initiation of gingivitis. Recognition of these relationships have been pivotal in establishing clinical practices in dentistry and have aided the discovery of therapeutic agents for preventative approaches such as triclosan, chlorhexidine, cetylpyridinium chloride and others. Despite these advances, it is clear that clinical measurements of gingivitis have some limitations and do not permit detection of the earliest events associated with gingivitis.

Described in the literature are studies on the role of the polymorphonuclear neutrophils (PMN) in the defense mechanisms during inflammatory conditions of the mouth (Kornman et al., 1997). While studies have primarily focused on the role of PMN in defense mechanisms those evaluating PMN in the oral cavity or in the saliva are lacking. In addition, there is not much information that describes the effects of specific oral hygiene formulations on the levels of PMN.

As with most medical conditions, it is desirable to detect oral inflammation early in order to start early treatment to avoid worsening the inflammation. This is especially true with respect to gingivitis and periodontitis, i.e. while the former is an undersirable condition, its effect can be reversed. In contrast, periodontitis is a more severe oral condition beyond gingivitis wherein the damage to the oral tissue is essentially irreversible.

Moreover, a complicating factor for determining the degree of oral inflammation for many patients around the world is limited access to professional dental care either by way of proximity to the dental care professional or due to the cost of the diagnosis and treatment method.

As such, there is still a need in the art for safe, non-invasive, facile and cost-effective means of monitoring the status of oral inflammation in a patient and/or monitoring the progress of an oral inflammation treatment.

BHADBHADE et al. ("Correlation between probing pocket depth and neutrophil counts in dental plaque, saliva, and gingival crevicular fluid"), QUINTESSENCE INTERNATIONAL (BERLIN, GERMANY : 1985) FEB 2012, (201202), vol. 43, no. 2, ISSN 1936-7163, pages 111 - 117) suggests that neutrophil play a critical role in the innate immune system. The neutrophil levels in, e.g., dental plaque, and saliva were investigated.

ADONOGIANAKI et al. ("Lactoferrin in the gingival crevice as a marker of polymorphonuclear leucocytes in periodontal diseases", JOURNAL OF CLINICAL PERIODONTOLOGY, (19930101), vol. 20, no. 1, ISSN 0303-6979, pages 26 - 31) discloses a study regarding lactoferrin levels in gingival crevicular fluid.

MARTINS et al. ("CORRELATION OF LACTOFERRIN WITH NEUTROPHILIC INFLAMMATION IN BODY FLUIDS", CLINICAL AND DIAGNOSTIC LABORATORY IMMUNOLOGY, AMERICAN SOCIETY FOR MICROBIOLOGY, US, (19951101), vol. 2, no. 6, ISSN 1071-412X, pages 763 - 765) refers to determining the usefulness of lactoferrin as marker of neutrophilic inflammation in different body fluids.

MC MURRAY et al. ("The relationship between elastase and lactoferrin in healthy, gingivitis and periodontitis sites", ORAL DISEASES, (19950901), vol. 1, no. 3, ISSN 1354-523X, pages 106 - 109) refers to comparing the relative amounts of elastase and lactoferrin in the gingival crevicular fluid.

ZIA et al ("Oral biomarkers in the diagnosis and progression of periodontal diseases", BIOLOGY AND MEDICINE, (20111023), vol. 3, no. 2, pages 45 - 52) refers to recent advances in the use of salivary and gingival crevicular fluid biomarker-based disease diagnostics that focus on the indentification of active periodontal disease.

ESTELA et al. ("Innovative study on lactoferrin in periodontal disease Papel de la lactoferrina en enfermedades periodontales", REVISTA ODONTOLÓGICA MEXICANA, (20110101), vol. 15, no. 4, pages 231 - 237) refers to lactoferrin for use in the prevention and treatement of periodontal diseases.

SUOMALAINEN et al. ("Peroxidases, lactoferrin and lysozyme in peripheral blood neutrophils, gingival crevicular fluid and whole saliva of patients with localized juvenile periodontitis.", ORAL DISEASES, (19960601), vol. 2, no. 2, ISSN 1354-523X, pages 129 - 1 34) examines the longitudinal association of selected non-immune anti-microbial host factors to the localized juvenile periodontitis disease status.

WASZKIEWICZ et al. ("Decrease in salivary lactoferrin output in chronically intoxicated alcohol-dependent patients", FOLIA HISTOCHEMICA ET CYTOBIOLOGICA, (20120705), vol. 50, no. 2, ISSN 0239-8508, pages 248 - 254) studies the effect of chronic alcohol intoxication on salivary lactoferrin concentration and output.

- Moosani et al. ("Evaluation of Oral Neutrophil Levels as a Quantitative Measure of Periodontal Inflammatory Load in Patients with Special Needs", 1 November 2012 (2012-11-01), Retrieved from the Internet: URL: https://tspace.library.utoronto.ca/bitstream/1807/33456/1/Moosani Anita 201211 MSc thesis.p df) refers to a study regarding the evaluation of oral neutrophil levels as a quantitative measure of periodontal inflammatory load in patients with special needs.

- Jentsch et al. ("Lactoferrin and other markers from gingival crevicular fluid and saliva before and after periodontal treatment", JOURNAL OF CLINICAL PERIODONTOLOGY, vol. 31, no. 7, 1 July 2004 (2004-07-01), pages 511-514) refers to a study for verifying (i) if crevicular fluid defence variables reflect the changes after surgical periodontal treatment and (ii) if they are in correspondence with changes of these variables in the unstimulated whole saliva.

- Scott et al. ("Neutrophils in Periodontal Inflammation" In: "Periodontal Disease", 1 January 2011 (2011-01-01), KARGER, Basel, vol. 15, pages 56-83) refers to an association between neutrophil infiltration into the periodontal tissues and the severity and progression of inflammatory periodontal diseases.

Bender et al. ("Novel rinse assay for the quantification of oral neutrophils and the monitoring of chronic periodontal disease", Journal of Periodontal Research, vol. 41, no. 3, 1 June 2006 (2006-06-01), pages 214-220) refers to a novel rinse assay for the quantifiation of oral neutrophils and the monitoring of chronic periodontal disease.

### BRIEF SUMMARY

It has been surprisingly discovered that the amount, number or level of polymorphonuclear leukocytes (PMN) or PMN indicator substance in a subject's saliva is correlated to the level or degree of oral inflammation in said subject. Accordingly, the present invention concerns a method of monitoring the effectiveness of oral inflammation treatment in a subject comprising (a) providing a test sample of oral material from said subject, (b) determining the amount of polymorphonuclear neutrophils, or polymorphonuclear neutrophil indicator substance in said test sample, (c) comparing the amount of polymorphonuclear neutrophil, or polymorphonuclear neutrophil indicator substance in the test sample to a control sample, wherein the control sample is a sample of an oral material from the same subject from a prior time, and wherein the subject has been treated with one or more antimicrobial agents. Furthermore, the invention refers to the use of a diagnostic kit for monitoring the effectiveness of oral inflammation treatment in a subject, wherein the use comprises (a) providing a test sample of oral material from said subject, (b) determining the amount of polymorphonuclear neutrophils, or polymorphonuclear neutrophil indicator substance in said test sample, (c) comparing the amount of polymorphonuclear neutrophil, or polymorphonuclear neutrophil indicator substance in the test sample to a control sample, wherein the control sample is a sample of an oral material from the same subject from a prior time, wherein the subject has been treated with one or more antimicrobial agents, and wherein the diagnostic kit comprises a saliva absorbent material which contains reagents to determine the presence and/or amount of polymorphonuclear neutrophil indicator substance together with instructions for application of the saliva absorbent material to the oral cavity and instructions for a treatment method based on the amount of polymorphonuclear neutrophil indicator substance detected.

The method and use of the invention are easy to implement at point of care settings, e.g., chair side, dental clinics, dental camps, and the like. Because of the facile, safe, and non-invasive nature of the method of the invention, in some embodiments the method can be carried out by the patient who can record/report the results.

### DETAILED DESCRIPTION

As used throughout, ranges are used as shorthand for describing each and every value that is within the range. Any value within the range can be selected as the terminus of the range. In the event of a conflict in a definition in the present disclosure and that of a cited reference, the present disclosure controls. Unless otherwise specified, all percentages and amounts expressed herein and elsewhere in the specification should be understood to refer to percentages by weight. All percentages expressed herein are on a weight by dry matter basis unless specifically stated otherwise.

In the context of the invention, the term "treating" or "treatment", as used herein, means reversing, alleviating, mitigating or inhibiting the progress of the disorder or condition to which such term applies, or one or more symptoms of such disorder or condition. As used herein and in the appended claims, the singular forms "a", "an", and "the" include plural reference unless the context clearly dictates otherwise.

The term "normal control" refers to a subject or pool of subjects that does not have oral inflammation.

The term "swab" refers to any device suitable for collecting oral fluid formed from an absorbent material. Preferably, the absorbent material is attached to a holder, but the term swab, as used herein, includes free standing devices made from absorbent material that may be placed in the mouth or mouth surface to absorb the saliva located therein.

The term "oral material" refers to any substance in the oral cavity that can be sampled, e.g., saliva, gingival crevicular fluid, oral scrapings from the tounge or cheek, gingival plaque, and the like. A preferred oral material for the methods of the invention is saliva.

As used herein, "measure" or "determine" refers to any qualitative or quantitative determinations.

In the methods of the invention, the subject can be any animal, and is preferably a mammal such as a human, mouse, rat, cat, dog, horse, cow, donkey, sheep, or pig. Humans are most preferred.

The method comprises obtaining a test sample of oral material such as saliva from the subject. The saliva can be obtained by any means suitable in the art. For example, various oral fluid collection devices and kits are known in the art and are commercially available. The oral fluid collection device can be comprised of or otherwise shaped or formed as particles, fibers, plates, and the like. Saliva can be collected by aspiration or absorption. Suitable oral fluid collection devices are commercially available from Oasis Diagnostics Corp., Vancouver, WA, such as Super-SAL™, VerOFy®, Versi-SAL®, and UltraSal-2™.

In some aspects, the oral material, e.g., saliva, is obtained by means of a device such as a swab. The swab can be comprised of any material suitable in the art such as alginate, sponge, capillary matrix, filter paper, calcium fibers, cellulose-based materials, or synthetic polymers such as a polyurethane, polyester, rayon, or a polymer of ethylene glycol and terephthalic acid. Polymers of ethylene glycol and terephthalic acid (i.e., polyethylene terephthalate) sold under the trade name DACRON® (DuPont) are particularly suited to the present invention. It is preferred that the swab not contain cotton or other natural cellulose-based materials, because cotton may be contaminated with metals from the soil in which the cotton plant was grown, thereby potentially contaminating the assay and producing inaccurate or false positive results. The absorbent material of the swab can be impregnated with salts or a hypertonic solution to facilitate absorption. The absorbent material can also be impregnated with a flavorant to make the sampling more pleasant to the subject. The swab can be on the end of a holder or an applicator made from plastic, wood, aluminum, and the like, with synthetic materials such as plastic being preferred. The swab can then placed in an aqueous solution, preferable sterile and buffered. The swab can be swirled, vortexed, etc. to dislodge the PMN and/or their derivatives or components from the swab material into the aqueous solution.

In another embodiment the saliva is obtained by expectoration. For example, a subject rinses the oral cavity with an aqueous solution, preferably sterile and/or buffered. Examples of buffered solutions include Hanks Balanced Salt solution, phosphate buffered saline, TRIS buffer, and the like. The contents of the oral cavity, i.e, salivary samples, are then expectorated into containers, preferable sterile.

The number of PMN can be determined in the samples via conventional techniques known in the art, e.g., via cell counters, microscopy, and the like. If microscopy is used, the salivary samples can then be concentrated, e.g., by centrifugation, filtration, etc. and then resuspended. Cell stains, conventional or fluorescent, such as acridine orange, and the like can be used to stain the PMN to make them more visible. In one embodiment the number of PMN can be determined by the methodology disclosed in the paper: Bender JS, Thang H, Glogauer M, (2006) "Novel rinse assay for the quantification of oral neutrophils and the monitoring of chronic periodontal disease", J. Periodontal Res. 2006, 41:214-220.

The time required to collect saliva from the subject can vary. In some aspects, the oral fluid collection device or buffered solution is to remain in the subject's mouth for at least about 5 seconds, or at least about 10 seconds, or at least about 15 seconds, or at least about 20 seconds, or at least about 30 seconds. In some aspects, the oral fluid collection device or buffer solution can remain in the subject's mouth for at least about 1 minute, or more.

The method of the invention is for monitoring the effectiveness of oral inflammation (such as gingivitis or periodontitis, and associated conditions, e.g., bad breadth or halitosis) treatment in a subject,.

The amount of PMN or PMN indicator substance in a test sample is compared to a control, wherein the control sample is a sample of an oral material from the same subject from a prior time, and wherein the subject is treated with one or more antimicrobial agents.

The method of the invention is to monitor the effectiveness of oral inflammation treatment and the control is a test sample taken from the same subject at a prior time, which can be before or after treatment is initiated. The results obtained from practicing the monitoring method of the invention can be used to adjust or modify the oral inflammation treatment, that is, change the practices, behavior, regimen, dose and/or substance, e.g., dentifrice or medicament, used to treat the inflammation.

The present invention encompasses measuring PMN indicator substances instead of measuring PMN directly. The amounts of PMN indicator substances are any substances that can be measured in oral materials such as saliva wherein the amount of substance is correlated with the amounts of PMN. Such PMN indicator substances include, for example, metabolites, components, enzymes, cytokines, PMN excretions, PMN breakdown products, and the like. Specific examples of PMN indicator substances include salivary lactoferrin, calprotectin, and salivary leukocyte esterase. In most embodiments, the correlation with PMN is positive, e.g., the greater the amount of lactoferrin and/or leukocyte esterase, the greater the amount of PMN. However, it is also contemplated that a negative correlation may exist, i.e., the greater the amount of a particular measured substance, the lesser amount of PMN. In either case, standards can be developed by techniques known in the art, e.g., development of data tables or standard curves, to compare the amount of a particular substance in the oral cavity to the presence, absence or severity of oral inflammation.

Assays are known in the art for determining the presence and/or amount of leukocyte esterase, calprotectin, and/or lactoferrin in biological samples. Such assays can be based on detecting reactions of the substance to be measured, or complexes formed therewith, by color changes, fluorescence, luminescence, radioisotopic detection, ELSIA, and the like.

U.S. Patent 5,776,780, discloses a reagent system for detecting and measuring leukocyte esterase. The color generating mechanism or indicator of the reagent system is the result of leukocyte esterase acting upon compatible esters. This ester/esterase reaction produces a relatively unstable indoxyl moiety that is oxidized to form an indigo color that is monitored by monochromatic spectrophotometry. The addition of dehydrogenase to the reagent will enhance the speed of reaction and completeness of the reaction of the indoxyl moiety. The dehydrogenase oxidizes the alcohol group on the indoxyl group and promotes formation of a ketone. This transitional indoxyl ketone radical enhances color development, specificity, and accuracy and sensitivity of the reaction. The reagent system may contain one or more of the following compounds, 2,4-dinitrophenylhydrazine, hydroxylamine, or semicarbizide, which in the presence of indoxide ketones will give color development that can be monitored at the same wavelength as the indigo. A further enhancement of the method concerning the indoxyl intermediate, is the addition of p-dimethylaminobenzaldehyde or p-nitro-benzenediazonium tetrafluroborate or other azo indicators.

In one embodiment, the invention provides a diagnostic kit for monitoring the effectiveness of oral inflammation treatment in a subject, wherein the use comprises (a) providing a test sample of oral material from said subject, (b) determining the amount of polymorphonuclear neutrophil, or polymorphonuclear neutrophil indicator substance in said test sample, (c) comparing the amount of polymorphonuclear neutrophil, or polymorphonuclear neutrophil indicator substance in the test sample to a control sample, wherein the control sample is a sample of an oral material from the same subject from a prior time, wherein the subject is treated with one or more antimicrobial agents, and wherein the diagnostic kit comprises a saliva absorbent material which contains reagents to determine the presence and/or amount of polymorphonuclear neutrophil indicator substance together with instructions for application of the saliva absorbent material to the oral cavity and instructions for a treatment method based on the amount of polymorphonuclear neutrophil indicator substance detected.

In one embodiment the means for determining production of the indoxyl moieties is by detecting a color change.

Assays for lactoferrin can be based on detecting antibody/antigen complexes. The diagnostic kit for determining the amount of PMN in saliva sample by determining the amount of lactoferrin in said sample specifically reactive with an antibody specific for lactoferrin, can comprise:
(a) a lactoferrin specific antibody;
(b) means for contacting the lactoferrin specific antibody with the sample to produce complexes comprising the lactoferrin specific antibody and any lactoferrin present in the sample; and
(c) means for measuring production of the complexes.

The lactoferring specific antibodies are commercially available or can be obtained though techniques known in the art, for example:
(a) administering lactoferrin to at least one mouse to produce at least one immunized mouse;
(b) removing B-lymphocytes from the at least one immunized mouse;
(c) fusing the B-lymphocytes from the at least one immunized mouse with myeloma cells, thereby producing hybridomas;
(d) cloning the hybridomas;
(e) selecting clones which produce anti-lactoferrin antibody;
(f) culturing the anti-lactoferrin antibody-producing clones; and then
(g) isolating anti-lactoferrin antibodies from the cultures.

In one embodiment, the lactoferrin is an antigen in immunoassays including enzyme-linked immunosorbent assays (ELISA), RIAs and other non-enzyme linked antibody binding assays or procedures known in the art for the detection of antibodies.

In ELISA assays, the lactoferrin specific antibody is immobilized onto a selected surface, for example, a surface capable of binding proteins such as filter paper or the wells of a polystyrene microtiter plate. After washing to remove incompletely adsorbed lactoferrin specific antibody, a nonspecific protein, such as a solution of bovine serum albumin (BSA) that is known to be antigenically neutral with regard to the test sample, may be bound to the selected surface. This allows for blocking of nonspecific adsorption sites on the immobilizing surface and thus reduces the background caused by nonspecific bindings onto the surface. The immobilizing surface is then contacted with a saliva sample to be tested in a manner conducive to immune complex (antigen/antibody) formation. This may include diluting the sample with diluents, such as solutions of BSA, bovine gamma globulin (BGG) and/or phosphate buffered saline (PBS)/Tween. The sample is then allowed to incubate for from about 2 to 4 hours, at temperatures such as of the order of about 25° to 37° C. Following incubation, the sample-contacted surface is washed to remove non-immunocomplexed material. The washing procedure may include washing with a solution, such as PBS/Tween or a borate buffer. Following formation of specific immunocomplexes between the test sample and the bound lactoferrin specific antibody, and subsequent washing, the occurrence, and even amount, of immunocomplex formation may be determined by subjecting the immunocomplex to a second antibody having specificity for the lactoferrin specific antibody.

If the lactoferrin specific antibody is of human origin, the second antibody can be an antibody having specificity for human immunoglobulins and in general IgG. To provide detecting means, the second antibody may have an associated activity such as an enzymatic activity that will generate, for example, a colour development upon incubating with an appropriate chromogenic substrate. Quantification may then be achieved by measuring the degree of colour generation using, for example, a visible spectra spectrophotometer. Modifications to this procedure can be made via techniques known in the art. For example, sandwich lateral flow chromatographic immunoassays can be employed.

In other embodiments, the assays for PMN indicator substances, such as lactoferrin, calprotectin and leukocyte esterase, can be miniaturized and/or streamlined. For example, the necessary reagents can be incorporated into a saliva absorbent material, such as a water absorbent material such as cellulose. The saliva absorbent material can be in the form of sheets or strips or be incorporated into devices such as a diptick that can be directly contacted with an oral material such as saliva. In such embodiments the strip or dipstick can be directly contacted with saliva in the oral cavity, thus obviating the need to collect a separate sample. Is such assays the presence and/or amount of PMN indicator substance can be determined by a color change and comparing the test color to a standard color chart.

Such assays are commercially available for detecting substances in urine or feces, e.g., OnSite FOB Hi Rapid Test-Cassette, available from CTK Biotech, Inc; Urinalysis Reagent Test Strips, available from Tigermedical; CLIA-URS-10 Urine Reagent Strips, available from CLIAwaived, Inc., calprotectin ELISA kits available from Alpha Laboratories such as CALcheck Blue™ and Quantum Blue®; and the like. Some commercially available assays have the capability of testing for PMN indicator substances such as leukocyte esterase, typically concomitantly for other substances as well, and can be used in the methods of the invention without modification. However, in some embodiments the assays are tailored to test for saliva and only for PMN indicator substances.

The level of lactoferrin in an oral material such as saliva is correlated with the amount of PMN in the oral material such as saliva. Thus, the method of the present invention can comprise determining the amount of lactoferrin in the subject(s) sample(s) at a prior time, and obtaining one or more samples from the test subject which is treated with one or more antimicrobial agents and determining the amount of lactoferrin in the test subject sample(s), and comparing the amount of lactoferrin in the normal sample(s) from said prior time to the amount of lactoferrin in the test sample(s).

That is, the invention concerns a method for monitoring the effectiveness of oral inflammation treatment in a subject having oral inflammation and which is treated with one or more antimicrobial agents, by comparing the level of lactoferrin in one or more oral material samples at a first time point (prior time) to the level of lactoferrin at a second time point; and correlating a decrease in the level of lactoferrin at the second time point as compared to the first time point with an improvement in the subject's disease state and, thus, improved effectiveness of oral inflammation treatment, and/or correlating an increase in the level of lactoferrin at the second time point as compared to the first time point with an increase in the severity of the subject's disease state, and thus reduced effectiveness of oral inflammation treatment.

The level of leukocyte esterase in an oral material such as saliva is correlated with the amount of PMN in the oral material such as saliva.

Thus, the invention concerns a method for monitoring the effectiveness of oral inflammation treatment, by comparing the level of leukocyte esterase in one or more oral material samples of a subject which is treated with one or more antimicrobial agents at a first time point to the level of leukocyte esterase at a second time point; and correlating a decrease in the level of leukocyte esterase at the second time point as compared to the first time point with an improvement in the subject's disease state and, thus, improved effectiveness of oral inflammation treatment, and/or correlating an increase in the level of leukocyte esterase at the second time point as compared to the first time point with an increase in the severity of the subject's disease state and, thus, reduced effectiveness of oral inflammation treatment.

The level of calprotectin in an oral material such as saliva is correlated with the amount of PMN in the oral material such as saliva. Thus, the present invention in another embodiment concerns a method for monitoring the effectiveness of oral inflammation treatment based on determining the levels of calprotectin.

The method of the invention can be used to initiate, change or modify oral inflammation treatments.

The step of the subject carrying out the selected treatment regimen is preferred, in one embodiment, in consultation with a dental professional.

The treatment regimen will vary depending on the result of the method of the invention. In some instances the comparison will show little or no inflammation, in which case the treatment regimen may comprise toothbrushing with a suitable dentrifrice once or twice per day. If the comparison indicates some degree of oral inflammation, then one or more of several types of treatment regimens may be selected, for example;
1. Toothbrushing more than twice per day, e.g., 3, 4, 5, or 6 times,
2. The use of antimicrobial agents. The antimicrobial agent can in incorporated into a dentifrice, e.g., toothpaste or mouthwash, or can be administered via other means, e.g., in tablet, suppository, transdermal patch, or injectable form. The amount administered will be an effective anti-microbial amount, i.e., an amount sufficient to eliminate or lessen the severity of the oral inflammation.
3. The use of antiflammatory agents. The antiinflammatory agent can in incorporated into a dentifrice, e.g., toothpaste or mouthwash, or can be administered via other means, e.g., in tablet, suppository, transdermal patch, or injectable form. The amount administered will be an effective anti-inflammatory amount, i.e., an amount sufficient to eliminate or lessen the severity of the oral inflammation.

The antimicrobial agent useful in the present invention is not particularly limited, and may be selected from halogenated diphenyl ether (e.g. triclosan), herbal extracts or essential oils (e.g., rosemary extract, thymol, menthol, eucalyptol, methyl salicylate), bisguanide antiseptics (e.g., chlorhexidine (CHX), alexidine, or octenidine), phenolic antiseptics, hexetidine, povidone iodine, delmopinol, salifluor, metal ions and their salts (e.g., zinc chloride, zinc lactate, zinc citrate, stannous fluoride, and stannous chloride), sanguinarine, propolis, oxygenating agents (e.g., hydrogen peroxide, buffered sodium peroxyborate, or peroxycarbonate), cetyl pyridinium chloride, magnolia extract, magnolol, honokiol, butyl magnolol, propyl honokiol, and mixtures thereof. Anti-attachment agents such as Solrol also can be included in dentifrices, as well as plaque dispersing agents such as enzymes (papain, glucoamylase, etc.).

Suitable anti-inflammatory agents include without limitation steroidal agents such as flucinolone and hydrocortisone, and nonsteroidal agents (NSAIDs) such as ketorolac, flurbiprofen, ibuprofen, naproxen, indomethacin, diclofenac, etodolac, indomethacin, sulindac, tolmetin, ketoprofen, fenoprofen, piroxicam, nabumetone, aspirin, diflunisal, meclofenamate, mefenamic acid, oxyphenbutazone and phenylbutazone.

Other known actives for oral care compositions can also be used in the treatment regimen such as a fluoride ion source (e.g. an amine fluoride, sodium fluoride, sodium monofluorophosphate, stannous fluoride, etc.) or a basic amino acid (e.g. arginine, an active ingredient in Colgate's Pro-Argin™ technology.

It is an advantage of the present invention that the facile, safe, and non-invasive nature of the methods of the invention allows for, in some embodiments, the methods to be carried out by the subject or patient who can record/report the results. On a routine basis patients need several motivated and compliance based approaches to help maintain and monitor their oral health status. The methods herein off a simple, cost-effective, rapid and easy means to perform routine evaluations of the oral health status in the privacy of their own homes. Thus, the methods herein provide suitable approaches that can be implementd in the home setting, i.e., are "at home" methods and kits. The methods allow subjects to monitor the effectiveness of oral inflammation treatment and maintain a periodic record of their findings that can be discussed and used in consultation with their professional dental provider. The methods can be used subsequent to a dental visit to monitor and evaluate their oral health status and seek or modulate dental care based on the results of the methods. In some embodiments the results can be computerized, electronically stored, and/or automatically submitted to a dental professional.

In the methods of the invention, the oral material samples such as saliva samples are compared on the same unit basis, e.g., 1, 0.1, 0.001 or 0.0001 ml. or other volumes of oral material as long as the volumes are the same.

The invention thus provides in one embodiment, a method (Method 1) of monitoring the effectiveness of oral inflammation treatment in a subject comprising (a) providing a test sample of oral material from said subject, (b) determining the amount of polymorphonuclear neutrophil, or polymorphonuclear neutrophil indicator substance in said test sample, (c) comparing the amount of polymorphonuclear neutrophil, or polymorphonuclear neutrophil indicator substance in the test sample to a control sample, wherein the control sample is a sample of an oral material from the same subject from a prior time, and wherein the subject is treated with one or more antimicrobial agents

Preferably, the invention provides
1.1. The method of any foregoing method wherein an increased level of PMN or PMN indicator substance in the test sample indicates ineffective oral inflammation treatment and a decreased level of PMN or PMN indicator substance in the test sample indicates effective oral inflammation treatment;
1.2. The method of any foregoing method wherein the oral inflammation is gingivitis;
1.3. The method of any foregoing claim wherein the oral inflammation is periodontitis;
1.4. The method of any foregoing method wherein an associated condition of the oral inflammation is halitosis;
1.5. The method of any foregoing method wherein the test sample is obtained with a swab;
1.6. The method of any foregoing method wherein the test sample is obtained by expectoration;
1.7. The method of any of the foregoing method wherein the amount of polymorphonuclear neutrophils is determined by microscopy;
1.8. The method of any of the foregoing method wherein the polymorphonuclear neutrophil indicator substance is lactoferrin;
1.9. The method of any foregoing method wherein the polymorphonuclear neutrophil indicator substance is leukocyte esterase;
1.10. The method of any foregoing method wherein the polymorphonuclear neutrophil indicator substance is calprotectin;
1.11. The method of any of the foregoing method wherein the amount of PMN indicator substance is determined by contacting saliva with a saliva absorbent material which contains the necessary reagents to determine the presence and/or amount of PMN indicator substance;
1.12. The method of the immediately preceding method wherein the presence and/or amount of PMN indicator substance is identified by a color change.

A diagnostic kit for use in the invention may compries a saliva absorbent material which contains the necessary reagents to determine the presence and/or amount of PMN indicator substance together with instructions for use.

The invention is illustrated by the following non-limiting examples.

### EXAMPLES

### Example 1:

Objective: This clinical study evaluated the levels of polymorphonuclear leukocytes (PMN), a type of immune cell in comparison to the clinical parameters of dental plaque (plaque index) and gingivitis (gingival Index) respectively.

Procedure: For this study, subjects were evaluated by these methods at Baseline, Visit 1 (7 days), and Visit 2 (14 days). For the study period, all subjects were provided a commercially available fluoride toothpaste for oral hygiene.

### Dental Plaque Assessment:

The dentition is disclosed with disclosing solution and plaque is scored at the disto-, mid-, mesio-buccal, and disto-, mid-, mesio-lingual surfaces of each tooth according to the criteria of the modified Quigley and Hein Index (Turesky et al., 1970 and Quigley & Hein, 1962), which is scored on a 0 to 5 scale. Subjectwise scores are calculated by summing all scores for all sites and dividing by the total number of sits scored.

### Gingivitis Assessment

The degree of gingivitis is scored at 6 sites (same sites listed above) of each tooth according to the criteria of the Gingival Index System (Löe and Silness, 1963), which is scored on a 0 to 3 scale. Subjectwise scores are calculated by summing all scores for all sites and dividing by the total number of sites scored.

### PMN Measurement

Prior to clinical assessments, subjects are instructed to rinse vigorously with Hank's Balanced Salt solution (Gibco® HBSS, Life Technologies) for 30 seconds and then expectorated into sterile tubes. Formaldehyde is added to the samples which are then centrifuged. The resulting pellets are resuspended and stained with acridine orange in a dark room at room temperature for 20 minutes prior to analysis by fluorescence microscopy.

Results: One hundred fifty-nine (159) adults (61 men and 98 women; age range 19-61 years) were enrolled in study.

Subject demographics are shown in Table 1. Summaries from clinical evaluations are shown in Tables 2 through 4 below.

**Table 1: Summary of Age and Sex Characteristics for enrolled subjects.**

| Number (Percent) of subjects | | | Age of subjects | |
|---|---|---|---|---|
| Male | Female | Total | Mean | Range |
| 61 (38.4) | 98 (61.6) | 159 (100) | 30.8 | 19-61 |

In order to compare PMN with Plaque Index and Gingival Index a graphical display and a corresponding correlation analysis was performed. Prior to analysis, due to the skewed distribution of the PMN scores, a log10 data transformation was employed. The analysis shows that the correlation coefficient of Log(PMN) with Plaque Index is 0.57 and the correlation coefficient of Log(PMN) with Gingival Index is 0.66. Both correlation coefficients are significantly (P<0.0001) different from zero.

The analysis above demonstrates that Log(PMN) is giving results that are strongly correlated with the Plaque Index and the Gingival Index. The final phase of the analysis is designed to assess the statistical sensitivity of Log(PMN) compared to the Plaque Index and the Gingival Index, with respect to the ability of the measure to detect changes from Baseline. Tables 2, 3, and 4 summarize the data that are analyzed and the results of the analysis.

In Table 2 the baseline data are summarized for each of the three indices.

**Table 2**

| Baseline | | |
|---|---|---|
| Plaque Index | Gingival Index | Log(PMN) |
| 2.88 ± 0.94 | 1.37 ± 0.82 | 5.28 ± 0.56 |

In Tables 3 and 4 the change from baseline data to visits 1 and 2, respectively, are summarized and the analysis is summarized through the t-Statistic and P-Value for the significance of the mean change from Baseline. The t-Statistic is an outcome measure of this analysis and is used to determine the P-value used to assess the statistical significance of the change from Baseline.

**Table 3**

| Change from Baseline to Visit 1 (Visit 1 - Baseline) | | | |
|---|---|---|---|
| | Plaque Index | Gingival Index | Log(PMN) |
| Mean Change ± Std. | -0.11 ± 0.40 | -0.25 ± 0.29 | -0.22 ± 0.45 |
| P-Value | 0.0007 | <0.0001 | <0.0001 |
| Absolute Value of t-Statistic | 3.47 | 10.66 | 6.19 |

**Table 4**

| Change from Baseline to Visit 2 (Visit 2 - Baseline) | | | |
|---|---|---|---|
| | Plaque Index | Gingival Index | Log(PMN) |
| Mean Change ± Std. | 0.02 ± 0.36 | -0.41 ± 0.39 | -0.27 ± 0.46 |
| P-Value | 0.5127 | <0.0001 | <0.0001 |
| Absolute Value of t-Statistic | 0.66 | 13.44 | 7.34 |

The summaries shown in Table 2 give the average and standard deviation of the Baseline value for each measure. In Tables 3 and 4 all analyses show statistically significant (p<0.0001) mean changes from Baseline except for the change between Visit 2 and Baseline for the Plaque Index. However, in both Tables 3 and 4 the largest t-statistic is associated with the Gingival Index. Since the larger the t-Statistic the greater the statistical sensitivity of the measure it can be concluded that in this study the Gingival Index has more statistical sensitivity than the other two measures in terms of detecting changes from baseline. The Log(PMN), while showing more statistical sensitivity than the Plaque Index, shows less sensitivity than the Gingival Index.

The population of the 159 adults evaluated could be categorized into discrete groups based on plaque index, gingival index and PMN counts with lower number indices being indicative of a healthier oral environment.

| Variable | Group | Count | Mean |
|---|---|---|---|
| Plaque index | A | 45 | 1.79 |
| | B | 11 | 1.99 |
| | C | 19 | 3.16 |
| | D | 38 | 3.35 |
| | E | 46 | 3.64 |
| Gingival index | A | 45 | 0.29 |
| | B | 11 | 0.65 |
| | C | 19 | 1.30 |
| | D | 38 | 1.79 |
| | E | 46 | 2.29 |
| PMN (Counts per ml) | A | 45 | 65556 |
| | B | 11 | 139545 |
| | C | 19 | 313487 |
| | D | 38 | 535230 |
| | E | 46 | 690435 |

### Example 2:

**Objective:** This clinical study evaluated the levels of polymorphonuclear leukocytes (PMN) in conjunction with clinical parameters i.e. dental plaque (PI), gingival inflammation (GI) and periodontal pocket probing depth (PD) amongst the following groups of subjects stratified on the basis of their initial clinical status:
- healthy: subjects with no gingivitis and in good oral health
- gingivitis: subjects with gingivitis based on clinical evaluation
- periodontal disease: subjects with periodontal disease based on clinical evaluation

Shown in table 5 are demographic characteristics of enrolled subjects and results from clinical evaluations (table 6) and levels of polymorphonuclear leukocytes (table 7) prior to and after 28 day use of the triclosan/copolymer toothpaste (Colgate® Total - triclosan and polymethylvinylether/maleic anhydride copolymer) for oral hygiene.
Procedures are described herein or are similar to those described in Example 1.

**Table 5: Demographics of enrolled subjects.**

| **Parameter** | **Healthy** | **Gingivitis** | **Periodontal disease** |
|---|---|---|---|
| Number of subjects | 17 | 17 | 13 |
| Mean Age | 29.87 | 30 | 47.08 |
| Age SD | 8.52 | 6.54 | 11.70 |
| Minimum Age | 21 | 22 | 26 |
| Maximum Age | 52 | 45 | 64 |
| Number of males | 6 | 12 | 7 |
| Number of females | 11 | 5 | 6 |

**Table 6: Results from clinical evaluations of subjects prior to and after 28 day use of the triclosan/copolymer toothpas**

| | | Before treatment | After treatment | |
|---|---|---|---|---|
| Population | Clinical assessment | Average scores | Average scores | p value |
| Healthy | Dental plaque (PI) | 0.77 | 0.45 | 0.000 |
| Healthy | Gingival score (GI) | 0.47 | 0.38 | 0.046 |
| Healthy | Pocket Depth (PD) | 2.36 | 2.10 | 0.000 |
| Gingivitis | Dental plaque (PI) | 1.35 | 0.68 | 0.000 |
| Gingivitis | Gingival score (GI) | 1.43 | 0.59 | 0.000 |
| Gingivitis | Pocket Depth (PD) | 2.67 | 2.25 | 0.000 |
| Periodontal Disease | Dental plaque (PI) | 1.75 | 0.94 | 0.000 |
| Periodontal Disease | Gingival score (GI) | 1.78 | 0.88 | 0.000 |
| Periodontal Disease | Pocket Depth (PD) | 3.58 | 2.65 | 0.000 |

Results indicate significant reductions in each evaluated clinical parameter for all groups of evaluated subjects.

**Table 7: Counts for polymorphonuclear leukocytes prior to and after 28 day use of the triclosan/copolymer dentifrice.**

| Population | Before treatment | After treatment | % reduction | Comparing before treatment to after treatment scores (p value) |
|---|---|---|---|---|
| | Average numbers of 9 PMN (X10⁴) Cells per ml | Average numbers of PMN (X10⁴) Cells per ml | | |
| Healthy | 1.84 | 1.313 | 28.6 | <0.05 (Significant) |
| Gingivitis | 6.56 | 2.813 | 57.1 | <0.05 (Significant) |
| Periodontitis | 28.37 | 16.5 | 41.8 | <0.05 (Significant) |

Results indicate significant reductions in PMN scores in each group of evaluated subjects.

### Example 3:

**Objective:** This clinical study evaluated the levels of polymorphonuclear leukocytes (PMN), amongst subjects assigned either a commercially available fluoride toothpaste or the triclosan/copolymer toothpaste for oral hygiene. Subjects were evaluated prior to and after 30 day use of each toothpaste.

Shown in table 8 are results from this study.

Procedures are as described herein or are similar to the prior examples.

**Table 8: Counts for polymorphonuclear leukocytes amongst subjects prior to and after 30 days use of a fluoride toothpaste or the triclosan/copolymer dentifrice.**

| Treatment group | # of subjects | Baseline score ± SEM | Day 30 score ± SEM | % reduction |
|---|---|---|---|---|
| Fluoride toothpaste (Crest) | 37 | 14.05 ± 2.06 | 11.65 ± 1.6 | 17.08 |
| Triclosan/copolymer | 37 | 20.03 ± 4 | 13.08 ± 2.85 | 34.69 |
| | t-Test Results | No significant differences between treatments at baseline. P value = 0.100 | Significant differences between treatments at Day 30. | |
| | | | P value 0.00014 | |

Results indicate significant reductions in PMN scores amongst those provided the triclosan/copolymer toothpaste for oral hygiene. A similar effect was not observed amongst those using the fluoride toothpaste.

### Example 4:

This clinical study evaluates the levels of poylmorphonuclear leukocytes (PMN), in conjunction with the following well accepted clinical and microbiological parameters prior to and after 2 week use of a 0.12% chlorhexidine (CHX) mouthrinse for oral hygiene:
- Clinical parameters:
   ∘ Malodor based on organoleptic and halimeter evaluations.
   ∘ An evaluation of tongue coating based on a tongue coat clinical index.
- Microbiological parameters:
   ∘ Total viable organisms of the dental plaque, saliva and tongue surface.
   ∘ Malodor organisms in the dental plaque, saliva and tongue surface.

Shown in table 9 are demographics of enrolled subjects.

**Table 9: Patient Demographics**

| Status | N | Mean Age |
|---|---|---|
| Control | 15 | 39.6 |
| Gingivitis | 21 | 44.76 |
| Halitosis | 18 | 54.72 |

The numbers of polymorphonuclear leukocytes (PMN) from all subjects prior to and after use of the CHX mouthinse are determined. Results indicate reductions in PMN scores for all groups of subjects after two week use of the CHX mouthrinse.

Summarized in tables 10-12 are results from clinical evaluations of subejcts by a halimeter, organoleptic scores and by the tongue coat index.

**Table 10: Halitosis evaluation (using a Halimeter)**

| Status | Mean baseline scores for halitosis | Mean halitosis scores after 2 week use of 0.12% chlorhexidine |
|---|---|---|
| Control | 79.53 | 52.87 |
| Gingivitis | 111.62 | 51 |
| Halitosis | 253.5 | 107.7 |

**Table 11: Halitosis evaluation (Organoleptic Scores)**

| Status | Mean baseline scores for halitosis | Mean halitosis scores after 2 week use of 0.12% chlorhexidine |
|---|---|---|
| Control | 1.467 | 0.6 |
| Gingivitis | 2.476 | 1.19 |
| Halitosis | 3.684 | 1.632 |

**Table 12: Tongue Coat Index**

| Status | Mean baseline scores | Mean scores after 2 week use of 0.12% chlorhexidine |
|---|---|---|
| Control | 1.667 | 0.667 |
| Gingivitis | 2.619 | 1.619 |
| Halitosis | 3.632 | 2.053 |

Results indicate significant reductions in each clinical score after subjects were assigned the CHX rinse.

Summarized in tables 13-18 are results from microbiological evaluations of subejcts for viable organisms in dental plaque, saliva and tongue surface in additiona o maloor organisms in each of these samples.

**Table 13: Total Viable Bacteria in Dental Plaque (Log CFU/ml)**

| Status | Mean baseline scores | Mean score after 2 week use of 0.12% chlorhexidine |
|---|---|---|
| Control | 7.18 | 5.86 |
| Gingivitis | 7.49 | 6.25 |
| Halitosis | 7.58 | 6.15 |

**Table 14: Viable Dental Plaque Malodor Bacteria (Log CFU/ml).**

| Status | Mean baseline scores | Mean scores after 2 week use of 0.12% chlorhexidine |
|---|---|---|
| Control | 6.7 | 5.71 |
| Gingivitis | 6.86 | 5.86 |
| Halitosis | 6.83 | 5.4 |

**Table 15: Total Viable Bacteria on Tongue Surface (Log CFU/ml).**

| Status | Mean baseline scores | Mean scores after 2 week use of 0.12% chlorhexidine |
|---|---|---|
| Control | 7.26 | 6.5 |
| Gingivitis | 7.63 | 6.67 |
| Halitosis | 7.72 | 6.83 |

**Table 16: Viable Tongue Surface Malodor Bacteria (Log CFU/ml)**

| Status | Mean baseline scores | Mean scores after 2 week use of 0.12% chlorhexidine |
|---|---|---|
| Control | 6.75 | 6.13 |
| Gingivitis | 6.95 | 6.25 |
| Halitosis | 7.18 | 6.09 |

**Table 17: Total Viable Salivary Bacteria (Log CFU/ml)**

| Status | Mean baseline scores | Mean scores after 2 week use of 0.12% chlorhexidine |
|---|---|---|
| Control | 7.81 | 6.97 |
| Gingivitis | 7.86 | 7.01 |
| Halitosis | 7.78 | 7.16 |

**Table 18: Viable Salivary Malodor Bacteria (Log CFU/ml)**

| Status | Mean baseline scores | Mean scores after 2 week use of 0.12% chlorhexidine |
|---|---|---|
| Control | 7.03 | 6.2 |
| Gingivitis | 7.09 | 6.09 |
| Halitosis | 7.1 | 6.13 |

Subjects demonstrated significant reductions in all types of organisms in each oral sample evaluated following the use of the CHX rinse.

### Example 5

This study determines how quickly subjects brushing with Total or fluoride toothpaste begin to show changes in oral PMN and clinical outcomes.

### Study design:

1. Subjects brushed with either a fluoride toothpaste or with the Total toothpaste.
2. Subjects were evaluated at study entry (baseline or prior to issuing any toothpaste) and were also evaluated after brushing with provided paste for 1 week and 2 weeks.

The following evaluations were conducted for all subjects at baseline and after 1 week and 2 week use of assigned toothpaste:
1. PMN
2. Clinical parameters (dental plaque index, gingivitis index, bleeding index).

**Table 19**

| **Summary of Age &Gender** | | | | | |
|---|---|---|---|---|---|
| **For Subjects Who Completed the Clinical Study** | | | | | |
| | Number of Subjects | | | Age³ | |
| Treatment | Male | Female | Total³ | Mean | Range |
| Test Toothpaste Group¹ | 6 | 19 | 25 | 44.8 | 19-70 |
| Control Toothpaste Group² | 7 | 18 | 25 | 42.7 | 19-65 |

| | | | | | |
|---|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY) 2. Colgate Cavity Protection Great Regular Flavor Toothpaste (Colgate-Palmolive Co., New York, NY) 3. No statistically significant difference was indicated between the treatment groups with respect to either gender or age. | | | | | |

**Table 20**

| **Subject Mean (SD) Polymorphonuclear Leukocytes (Log Cells/ml) Samples at Baseline. 1-Week and 2-Weeks** | | | | | |
|---|---|---|---|---|---|
| **For Subjects Who Completed the Clinical Study** | | | | | |
| Parameter | Treatment | n | Baseline Summary (Mean ± S.D.) | 1-Week Summary (Mean ± S.D.) | 2-Week Summary (Mean ± S.D.) |
| Polymorphonuclear Leukocytes (Log Cells/ml) | Test Toothpaste Group¹ | 25 | 5.17 ± 0.32 | 4.93 ± 0.23 | 4.77 ± 0.28 |
| | Control Toothpaste Group² | 25 | 5.08 ± 0.40 | 5.08 ± 0.36 | 5.11 ± 0.38 |

| | | | | | |
|---|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY). 2. Colgate Cavity Protection Great Regular Flavor Toothpaste (Colgate-Palmolive Co., New York, NY). 3. No statistically significant difference was indicated between the two treatment groups at baseline with respect to polymorphonuclear leukocytes (Log Cell/ml) samples. | | | | | |

**Table 21**

| **Subject Mean (SD) Gingival Index, Bleeding Index, Plaque Index Scores and Pocket Depth at Baseline and 2-Weeks** | | | | |
|---|---|---|---|---|
| **For Subjects Who Completed the Clinical Study** | | | | |
| Parameter | Treatment | n | Baseline Summary (Mean ± S.D.) | 2-Week Summary (Mean ± S.D.) |
| Gingival Index | Test Toothpaste Group¹ | 25 | 1.42 ± 0.32 | 1.01 ± 0.35 |
| | Control Toothpaste Group² | 25 | 1.38 ± 0.26 | 1.32 ± 0.25 |
| Bleeding Index | Test Toothpaste Group¹ | 25 | 0.55 ±0.22 | 0.35 ± 0.25 |
| | Control Toothpaste Group² | 25 | 0.65 ± 0.33 | 0.59 ± 0.30 |
| Plaque Index | Test Toothpaste Group¹ | 25 | 2.17 ± 0.57 | 1.81 ± 0.49 |
| | Control Toothpaste Group² | 25 | 2.16 ± 0.51 | 2.02 ± 0.49 |
| Pocket Depth | Test Toothpaste Group¹ | 25 | 2.18 ± 0.46 | 1.92 ± 0.43 |
| | Control Toothpaste Group² | 25 | 2.06 ± 0.41 | 2.02 ± 0.38 |

| | | | | |
|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY). 2. Colgate Cavity Protection Great Regular Flavor Toothpaste (Colgate-Palmolive Co., New York, NY). 3. No statistically significant difference was indicated among the two treatment groups at baseline with respect to gingival index, bleeding index, plaque index scores and pocket depth (mm). | | | | |

**Table 22**

| **Baseline-Adjusted Subject Mean (SE) Polymorphonuclear Leukocytes (Log Cells/ml) Samples at 1-Week** | | | | | | | | | |
|---|---|---|---|---|---|---|---|---|---|
| **For Subjects Who Completed the Clinical Study** | | | | | | | | | |
| Index | Treatment | n | 1-Week Summary (Mean ± S.E.) | | | Within Treatment Analysis | | Between-Treatment Comparisons | |
| | | | | | | Reduction³ | Sig.⁴ | Difference⁵ | Siq.⁶ |
| Polymorphonuclear Leukocytes (Log Cells/ml) | Test Toothpaste Group¹ | 25 | 4.91 | ± | 0.05 | 38.3% | P=0.001 | 35.4% | P=0.009 |
| | Control Toothpaste Group² | 25 | 5.10 | ± | 0.05 | 4.5% | P=0.752 | | |

| | | | | | | | | | |
|---|---|---|---|---|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY). 2. Colgate Cavity Protection Great Regular Flavor Toothpaste (Colgate-Palmolive Co., New York, NY). 3. Reduction exhibited by the 1-week mean relative to the baseline mean. A positive value indicates a reduction in polymorphonuclear leukocytes (Log Cells/ml) at the 1-week examination. 4. Significance of paired t-test comparing the baseline and 1-week examinations. 5. Difference between the 1-week means expressed as a reduction of the 1-week mean for the Control Toothpaste Group. A positive value indicates a reduction in polymorphonuclear leukocytes (Log Cells/ml) for the Test Toothpaste Group relative to Control Toothpaste Group. 6. Significance of ANCOVA comparison of baseline-adjusted means. | | | | | | | | | |

**Table 23**

| **Baseline-Adjusted Subject Mean (SE) Polymorphonuclear Leukocytes (Log Cells/ml) Samples at 2-Weeks** | | | | | | | | | |
|---|---|---|---|---|---|---|---|---|---|
| **For Subjects Who Completed the Clinical Study** | | | | | | | | | |
| Index | Treatment | n | 2-Week Summary (Mean ± S.E.) | | | Within Treatment Analysis | | Between-Treatment Comparisons | |
| | | | | | | Reduction³ | Sig.⁴ | Difference⁵ | Siq.⁶ |
| Polymorphonuc lear Leukocytes (Log Cells/ml) | Test Toothpaste Group¹ | 25 | 4.75 | ± | 0.05 | 57.3% | P<0.001 | 59.3% | P<0.001 |
| | Control Toothpaste Group² | 25 | 5.14 | ± | 0.05 | -4.7% | P=815 | | |

| | | | | | | | | | |
|---|---|---|---|---|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY). 2. Colgate Cavity Protection Great Regular Flavor Toothpaste (Colgate-Palmolive Co., New York, NY). 3. Reduction exhibited by the 2-week mean relative to the baseline mean. A positive value indicates a reduction in polymorphonuclear leukocytes (Log Cells/ml) at the 2-week examination. 4. Significance of paired t-test comparing the baseline and 2-week examinations. 5. Difference between the 2-week means expressed as a reduction of the 2-week mean for the Control Toothpaste Group. A positive value indicates a reduction in polymorphonuclear leukocytes (Log Cells/ml) for the Test Toothpaste Group relative to Control Toothpaste Group. 6. Significance of ANCOVA comparison of baseline-adjusted means. | | | | | | | | | |

**Table 24**

| **Baseline-Adjusted Subject Mean (SE) Gingival Index, Bleeding Index, Plaque Index Scores and Pocket Depth** | | | | | | | | | |
|---|---|---|---|---|---|---|---|---|---|
| **For Subjects Who Completed the Clinical Study** | | | | | | | | | |
| | Treatment | N | 2-Week Summary (Mean ± S.E.) | | | Within Treatment Analysis | | Between-Treatment Comparisons | |
| | | | | | | Reduction³ | Sig.⁴ | Difference⁵ | Sig.⁶ |
| Gingival Index | Test Toothpaste Group¹ | 25 | 0.99 | ± | 0.05 | 29.3% | P<0.001 | 25.6% | P<0.001 |
| | Con Index trol Toothpaste Group² | 25 | 1.33 | ± | 0.05 | 5.0% | P=0.008 | | |
| Bleeding Index | Test Toothpaste Group¹ | 25 | 0.39 | ± | 0.03 | 35.0% | P<0.001 | 29.1% | P<0.001 |
| | Control Toothpaste Group² | 25 | 0.55 | ± | 0.03 | 8.3% | P=0.045 | | |
| Plaque Index | Test Toothpaste Group¹ | 25 | 1.80 | ± | 0.05 | 16.7% | P<0.001 | 11.3% | P=0.004 |
| | Control Toothpaste Group² | 25 | 2.03 | ± | 0.05 | 6.0% | P<0.001 | | |
| Pocket Depth (mm) | Test Toothpaste Group¹ | 25 | 1.87 | ± | 0.04 | 0.25mm | P<0.001 | 0.2mm | P=0.001 |
| | Control Toothpaste Group² | 25 | 2.07 | ± | 0.04 | 0.05mm | P=0.218 | | |

| | | | | | | | | | |
|---|---|---|---|---|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY). 2. Colgate Cavity Protection Great Regular Flavor Toothpaste (Colgate-Palmolive Co., New York, NY). 3. Reduction exhibited by the 2-week mean relative to the baseline mean. A positive value indicates a reduction in index scores at the 2-week examination. 4. Significance of paired t-test comparing the baseline and 2-week examinations. 5. Difference between the 2-week means expressed as a reduction of the 2-week mean for the Control Toothpaste Group. A positive value indicates a reduction in index scores for the Test Toothpaste Group relative to Control Toothpaste Group. 6. Significance of ANCOVA comparison of baseline-adjusted means. | | | | | | | | | |

### Results Summary:

- subjects brushing with Total begin to show reductions in PMN after only one week use of this toothpaste.
- additional reductions in PMN amongst subjects brushing with Total in the second week of use. In other words, the effects of Total get better from the week 1 to week 2.

### Significance of results:

1. PMN (or neutrophil) changes commence in the initial days following use of an effective toothpaste (Total).
2. Furthermore, the % changes for PMN are higher for Total than a fluoride toothpaste.
3. The % differences for PMN in the Total group are far higher than those for conventional clinical indices i.e. gingivitis index, bleeding index and plaque index.

### Example 6

Study Objective: Determine how quickly subjects brushing with Total or fluoride toothpaste begin to show changes in oral PMN and clinical outcomes.

### Study design:

1. Subjects brushed with either a fluoride toothpaste or with the Total toothpaste.
2. Subjects were evaluated at study entry (baseline or prior to issuing any toothpaste) and were also evaluated after brushing with provided paste for 1 week and 2 weeks.

The following evaluations were conducted for all subjects at baseline and after 1 week and 2 week use of assigned toothpaste:
1. PMN
2. Clinical parameters (dental plaque index, gingivitis index, bleeding index).

The results are in the tables below.

### Conclusions

- subjects brushing with Total begin to show reductions in PMN after only one week use of this toothpaste.
- additional reductions in PMN amongst subjects brushing with Total in the second week of use, i.e. the effects of Total get better from the week 1 to week 2.

**Table 25**

| Summary of Age &Gender | | | | | |
|---|---|---|---|---|---|
| For Subjects Who Completed the Clinical Study | | | | | |
| | Number of Subjects | | | Age³ | |
| Treatment | Male | Female | Total³ | Mean | Range |
| Test Toothpaste Group¹ | 6 | 19 | 25 | 44.8 | 19-70 |
| Control Toothpaste Group² | 7 | 18 | 25 | 42.7 | 19-65 |

| | | | | | |
|---|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY) 2. Colgate Cavity Protection Great Regular Flavor Toothpaste (Colgate-Palmolive Co., New York, NY) 3. No statistically significant difference was indicated between the treatment groups with respect to either gender or age. | | | | | |

**Table 26**

| Subject Mean (SD) Polymorphonuclear Leukocytes (Log Cells/ml) Samples at **Baseline. 1-Week and 2-Weeks** | | | | | |
|---|---|---|---|---|---|
| For Subjects Who Completed the Clinical Study | | | | | |
| Parameter | Treatment | n | Baseline Summary (Mean ± S.D.) | 1-Week Summary (Mean ± S.D.) | 2-Week Summary (Mean ± S.D.) |
| Polymorphonuclear Leukocytes (Log Cells/ml) | Test Toothpaste Group¹ | 25 | 5.17 ± 0.32 | 4.93 ± 0.23 | 4.77 ± 0.28 |
| | Control Toothpaste Group² | 25 | 5.08 ± 0.40 | 5.08 ± 0.36 | 5.11 ± 0.38 |

| | | | | | |
|---|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY). 2. Colgate Cavity Protection Great Regular Flavor Toothpaste (Colgate-Palmolive Co., New York, NY). 3. No statistically significant difference was indicated between the two treatment groups at baseline with respect to polymorphonuclear leukocytes (Log Cell/ml) samples. | | | | | |

**Table 27**

| Subject Mean (SD) Gingival Index, Bleeding Index, Plaque Index Scores and Pocket Depth at **Baseline and 2-Weeks** | | | | |
|---|---|---|---|---|
| For Subjects Who Completed the Clinical Study | | | | |
| Parameter | Treatment | n | Baseline Summary (Mean ± S.D.) | 2-Week Summary (Mean ± S.D.) |
| Gingival Index | Test Toothpaste Group¹ | 25 | 1.42 ± 0.32 | 1.01 ± 0.35 |
| | Control Toothpaste Group² | 25 | 1.38 ± 0.26 | 1.32 ± 0.25 |
| Bleeding Index | Test Toothpaste Group¹ | 25 | 0.55 ± 0.22 | 0.35 ± 0.25 |
| | Control Toothpaste Group² | 25 | 0.65 ± 0.33 | 0.59 ± 0.30 |
| Plaque Index | Test Toothpaste Group¹ | 25 | 2.17 ± 0.57 | 1.81 ± 0.49 |
| | Control Toothpaste Group² | 25 | 2.16 ± 0.51 | 2.02 ± 0.49 |
| Pocket Depth | Test Toothpaste Group¹ | 25 | 2.18 ± 0.46 | 1.92 ± 0.43 |
| | Control Toothpaste Group² | 25 | 2.06 ± 0.41 | 2.02 ± 0.38 |

| | | | | |
|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY). 2. Colgate Cavity Protection Great Regular Flavor Toothpaste (Colgate-Palmolive Co., New York, NY). 3. No statistically significant difference was indicated among the two treatment groups at baseline with respect to gingival index, bleeding index, plaque index scores and pocket depth (mm). | | | | |

**Table 28**

| Baseline-Adjusted Subject Mean (SE) Polymorphonuclear Leukocytes (Log Cells/ml) Samples at **1-Week** | | | | | | | | | |
|---|---|---|---|---|---|---|---|---|---|
| For Subjects Who Completed the Clinical Study | | | | | | | | | |
| Index | Treatment | n | 1-Week Summary (Mean ± S.E.) | | | Within Treatment Analysis | | Between-Treatment Comparisons | |
| | | | | | | Reduction³ | Sig.⁴ | Difference⁵ | Siq.⁶ |
| Polymorphonuclear | Test Toothpaste Group¹ | 25 | 4.91 | ± | 0.05 | 38.3% | P=0.001 | 35.4% | P=0.0 09 |
| Leukocytes (Log Cells/ml) | Control Toothpaste Group² | 25 | 5.10 | ± | 0.05 | 4.5% | P=0.752 | | |

| | | | | | | | | | |
|---|---|---|---|---|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY). 2. Colgate Cavity Protection Great Regular Flavor Toothpaste (Colgate-Palmolive Co., New York, NY). 3. Reduction exhibited by the 1-week mean relative to the baseline mean. A positive value indicates a reduction in polymorphonuclear leukocytes (Log Cells/ml) at the 1-week examination. 4. Significance of paired t-test comparing the baseline and 1-week examinations. 5. Difference between the 1-week means expressed as a reduction of the 1-week mean for the Control Toothpaste Group. A positive value indicates a reduction in polymorphonuclear leukocytes (Log Cells/ml) for the Test Toothpaste Group relative to Control Toothpaste Group. 6. Significance of ANCOVA comparison of baseline-adjusted means. | | | | | | | | | |

**Table 29**

| Baseline-Adjusted Subject Mean (SE) Polymorphonuclear Leukocytes (Log Cells/ml) Samples at **2-Weeks** | | | | | | | | | |
|---|---|---|---|---|---|---|---|---|---|
| For Sub ects Who Completed the Clinical Study | | | | | | | | | |
| Index | Treatment | n | 2-Week Summary (Mean ± S.E.) | | | Within Treatment Analysis | | Between-Treatment Comparisons | |
| | | | | | | Reduction³ | Sig.⁴ | Diff.⁵ | Sig.⁶ |
| Polymorphonuclear Leukocytes (Log Cells/ml) | Test Toothpaste Group¹ | 25 | 4.75 | ± | 0.05 | 57.3% | P<0.001 | 59.3% | P<0.001 |
| | Control Toothpaste Group² | 25 | 5.14 | ± | 0.05 | -4.7% | P=815 | | |

| | | | | | | | | | |
|---|---|---|---|---|---|---|---|---|---|
| 1. Dentifrice formulation containing 0.3% triclosan, 2.0% copolymer, 0.243% sodium fluoride in a silica base (Colgate-Palmolive Co., New York, NY). 2. Colgate Cavity Protection Great Regular Flavor Toothpaste (Colgate-Palmolive Co., New York, NY). 3. Reduction exhibited by the 2-week mean relative to the baseline mean. A positive value indicates a reduction in polymorphonuclear leukocytes (Log Cells/ml) at the 2-week examination. 4. Significance of paired t-test comparing the baseline and 2-week examinations. 5. Difference between the 2-week means expressed as a reduction of the 2-week mean for the Control Toothpaste Group. A positive value indicates a reduction in polymorphonuclear leukocytes (Log Cells/ml) for the Test Toothpaste Group relative to Control Toothpaste Group. 6. Significance of ANCOVA comparison of baseline-adjusted means. | | | | | | | | | |

**Table 30**

| Baseline-Adjusted Subject Mean (SE) Gingival Index, Bleeding Index, Plaque Index Scores and Pocket Depth | | | | | | | | | |
|---|---|---|---|---|---|---|---|---|---|
| For Subjects Who Completed the Clinical Study | | | | | | | | | |
| Index | Treatment | n | 2-Week Summary (Mean ± S.E.) | | | Within Treatment Analysis | | Between-Treatment Comparisons | |
| | | | | | | Reduction³ | Sig.⁴ | Difference⁵ | Siq.⁶ |
| Gingival Index | Test Toothpaste Group¹ | 25 | 0.99 | ± | 0.05 | 29.3% | P<0.001 | 25.6% | P<0.001 |
| | Control Toothpaste Group² | 25 | 1.33 | ± | 0.05 | 5.0% | P=0.008 | | |
| Bleeding Index | Test Toothpaste Group¹ | 25 | 0.39 | ± | 0.03 | 35.0% | P<0.001 | 29.1% | P<0.001 |
| | Control Toothpaste Group² | 25 | 0.55 | ± | 0.03 | 8.3% | P=0.045 | | |
| Plaque Index | Test Toothpaste Group¹ | 25 | 1.80 | ± | 0.05 | 16.7% | P<0.001 | 11.3% | P=0.004 |
| | Control Toothpaste Group² | 25 | 2.03 | ± | 0.05 | 6.0% | P<0.001 | | |
| Pocket Depth (mm) | Test Toothpaste Group¹ | 25 | 1.87 | ± | 0.04 | 0.25mm | P<0.001 | 0.2mm | P=0.001 |
| | Control Toothpaste Group² | 25 | 2.07 | ± | 0.04 | 0.05mm | P=0.218 | | |

| | | | | | | | | | |
|---|---|---|---|---|---|---|---|---|---|
| 1. Dentifrice formulation containing 0.3% triclosan, 2.0% copolymer (polymethylvinylether/maleic anhydride copolymer), 0.243% sodium fluoride in a silica base (Colgate-Palmolive Co., New York, NY). 2. Colgate Cavity Protection Great Regular Flavor Toothpaste (Colgate-Palmolive Co., New York, NY). 3. Reduction exhibited by the 2-week mean relative to the baseline mean. A positive value indicates a reduction in index scores at the 2-week examination. 4. Significance of paired t-test comparing the baseline and 2-week examinations. 5. Difference between the 2-week means expressed as a reduction of the 2-week mean for the Control Toothpaste Group. A positive value indicates a reduction in index scores for the Test Toothpaste Group relative to Control Toothpaste Group. 6. Significance of ANCOVA comparison of baseline-adjusted means. | | | | | | | | | |

## Claims

1. A method of monitoring the effectiveness of oral inflammation treatment in a subject comprising (a) providing a test sample of oral material from said subject, (b) determining the amount of polymorphonuclear neutrophils, or polymorphonuclear neutrophil indicator substance in said test sample, (c) comparing the amount of polymorphonuclear neutrophil, or polymorphonuclear neutrophil indicator substance in the test sample to a control sample, wherein the control sample is a sample of an oral material from the same subject from a prior time, and wherein the subject has been treated with one or more antimicrobial agents.

2. The method of claim 1 wherein the oral material is saliva.

3. The method of claim 1 or 2 wherein an increased level of polymorphonuclear neutrophil indicator substance in the test sample indicates ineffective oral inflammation treatment and a decreased level of polymorphonuclear neutrophil indicator substance in the test sample indicates effective oral inflammation treatment.

4. The method of any foregoing claims 1-3 wherein the oral inflammation is gingivitis, periodontitis, or halitosis.

5. The method of any foregoing claims 1-4 wherein the test sample is obtained with a swab.

6. The method of any foregoing claims 1-4 wherein the test sample is obtained by expectoration.

7. The method of any of the foregoing claims 1-6 wherein the polymorphonuclear neutrophil indicator substance is lactoferrin.

8. The method of any foregoing claims 1-6 wherein the polymorphonuclear neutrophil indicator substance is leukocyte esterase.

9. The method of any foregoing claims 1-6 wherein the polymorphonuclear neutrophil indicator substance is calprotectin.

10. The method of any foregoing claims 1-9 wherein the oral material is contacted with an absorbent material containing reagents to determine the presence or amount of polymorphonuclear neutrophil indicator substance.

11. The method of claim 10 wherein the absorbent material is a strip or in a dipstick.

12. The method of any foregoing claims 1-11 wherein the antimicrobial agent is selected from the group consisting of halogenated diphenyl ether, triclosan, herbal extracts or essential oils, rosemary extract, thymol, menthol, eucalyptol, methyl salicylate, bisguanide antiseptics, chlorhexidine, alexidine, or octenidine, phenolic antiseptics, hexetidine, povidone iodine, delmopinol, salifluor, metal ions and their salts, zinc chloride, zinc lactate, zinc citrate, stannous fluoride, and stannous chloride, sanguinarine, propolis, oxygenating agents, hydrogen peroxide, buffered sodium peroxyborate, or peroxycarbonate, cetyl pyridinium chloride, magnolia extract, magnolol, honokiol, butyl magnolol, propyl honokiol, and mixtures thereof.

13. Use of a diagnostic kit for monitoring the effectiveness of oral inflammation treatment in a subject, wherein the use comprises (a) providing a test sample of oral material from said subject, (b) determining the amount of polymorphonuclear neutrophils, or polymorphonuclear neutrophil indicator substance in said test sample, (c) comparing the amount of polymorphonuclear neutrophil, or polymorphonuclear neutrophil indicator substance in the test sample to a control sample, wherein the control sample is a sample of an oral material from the same subject from a prior time, wherein the subject has been treated with one or more antimicrobial agents, and wherein the diagnostic kit comprises a saliva absorbent material which contains reagents to determine the presence and/or amount of polymorphonuclear neutrophil indicator substance together with instructions for application of the saliva absorbent material to the oral cavity and instructions for a treatment method based on the amount of polymorphonuclear neutrophil indicator substance detected.

14. The use according to claim 13, wherein the instructions for application of the saliva absorbent material to the oral cavity comprise:
(a) obtaining a test sample of oral material from said subject,
(b) determining the amount of polymorphonuclear neutrophil indicator substance in said test sample,
(c) comparing the amount of polymorphonuclear neutrophil indicator substance in the test sample to a control sample.

15. The use according to claim 13 or 14 wherein the antimicrobial agent is selected from the group consisting of halogenated diphenyl ether, triclosan, herbal extracts or essential oils, rosemary extract, thymol, menthol, eucalyptol, methyl salicylate), bisguanide antiseptics, chlorhexidine, alexidine, or octenidine), phenolic antiseptics, hexetidine, povidone iodine, delmopinol, salifluor, metal ions and their salts, zinc chloride, zinc lactate, zinc citrate, stannous fluoride, and stannous chloride, sanguinarine, propolis, oxygenating agents, hydrogen peroxide, buffered sodium peroxyborate, or peroxycarbonate, cetyl pyridinium chloride, magnolia extract, magnolol, honokiol, butyl magnolol, propyl honokiol, and mixtures thereof.

## Patentansprüche

1. Verfahren zur Überwachung der Wirksamkeit einer Behandlung einer Mundentzündung an einem Subjekt, umfassend (a) Bereitstellen einer Testprobe von Material aus dem Mund des Subjekts, (b) Bestimmen der Menge an polymorphkernigen Neutrophilen oder einer Indikatorsubstanz für polymorphkernige Neutrophile in der Testprobe, (c) Vergleichen der Menge an polymorphkernigen Neutrophilen oder der Indikatorsubstanz für polymorphkernige Neutrophile in der Testprobe mit einer Kontrollprobe, wobei die Kontrollprobe eine Probe eines Materials aus dem Mund desselben Subjekts zu einem früheren Zeitpunkt ist, und wobei das Subjekt mit einem oder mehreren antimikrobiellen Mitteln behandelt wurde.

2. Verfahren nach Anspruch 1, wobei das Material aus dem Mund Speichel ist.

3. Verfahren nach Anspruch 1 oder 2, wobei ein erhöhtes Ausmaß von Indikatorsubstanz für polymorphkernige Neutrophile in der Testprobe eine nicht wirksame Behandlung einer Mundentzündung anzeigt und ein verringertes Ausmaß von Indikatorsubstanz für polymorphkernige Neutrophile in der Testprobe eine wirksame Behandlung einer Mundentzündung anzeigt.

4. Verfahren nach einem der vorhergehenden Ansprüche 1-3, wobei die Mundentzündung Zahnfleischentzündung, Parodontitis oder Mundgeruch ist.

5. Verfahren nach einem der vorhergehenden Ansprüche 1-4, wobei die Testprobe mit einem Tupfer gewonnen wird.

6. Verfahren nach einem der vorhergehenden Ansprüche 1-4, wobei die Testprobe durch Expektoration gewonnen wird.

7. Verfahren nach einem der vorhergehenden Ansprüche 1-6, wobei die Indikatorsubstanz für polymorphkernige Neutrophile Lactoferrin ist.

8. Verfahren nach einem der vorhergehenden Ansprüche 1-6, wobei die Indikatorsubstanz für polymorphkernige Neutrophile Leukozyten-Esterase ist.

9. Verfahren nach einem der vorhergehenden Ansprüche 1-6, wobei die Indikatorsubstanz für polymorphkernige Neutrophile Calprotectin ist.

10. Verfahren nach einem der vorhergehenden Ansprüche 1-9, wobei das Material aus dem Mund mit einem absorbierenden Material in Kontakt gebracht wird, das Reagenzien zum Bestimmen der Anwesenheit von oder Menge an einer Indikatorsubstanz für polymorphkernige Neutrophile enthält.

11. Verfahren nach Anspruch 10, wobei das absorbierende Material ein Streifen ist oder sich in einem Teststäbchen befindet.

12. Verfahren nach einem der vorhergehenden Ansprüche 1-11, wobei das antimikrobielle Mittel ausgewählt ist aus der Gruppe bestehend aus halogeniertem Diphenylether, Triclosan, Kräuterextrakten oder ätherischen Ölen, Rosmarinextrakt, Thymol, Menthol, Eukalyptol, Methylsalicylat, Bisguanid-Antiseptika, Chlorhexidin, Alexidin oder Octenidin, Phenol-Antiseptika, Hexetidin, Povidoniodin, Delmopinol, Salifluor, Metallionen und deren Salzen, Zinkchlorid, Zinklactat, Zinkcitrat, Zinn(II)-fluorid und Zinn(II)-chlorid, Sanguinarin, Propolis, oxygenierenden Mitteln, Wasserstoffperoxid, gepuffertem Natriumperoxyborat oder Peroxycarbonat, Cetylpyridiniumchlorid, Magnolienextrakt, Magnolol, Honokiol, Butylmagnolol, Propylhonokiol und Gemischen davon.

13. Verwendung eines Diagnose-Kits zur Überwachung der Wirksamkeit einer Behandlung einer Mundentzündung an einem Subjekt, wobei die Verwendung umfasst: (a) Bereitstellen einer Testprobe von Material aus dem Mund des Subjekts, (b) Bestimmen der Menge an polymorphkernigen Neutrophilen oder einer Indikatorsubstanz für polymorphkernige Neutrophile in der Testprobe, (c) Vergleichen der Menge an polymorphkernigen Neutrophilen oder der Indikatorsubstanz für polymorphkernige Neutrophile in der Testprobe mit einer Kontrollprobe, wobei die Kontrollprobe eine Probe eines Materials aus dem Mund desselben Subjekts zu einem früheren Zeitpunkt ist, und wobei das Subjekt mit einem oder mehreren antimikrobiellen Mitteln behandelt wurde, und wobei das Diagnose-Kit ein Speichel absorbierendes Material umfasst, das Reagenzien zum Bestimmen der Anwesenheit von und/oder Menge an einer Indikatorsubstanz für polymorphkernige Neutrophile zusammen mit Anweisungen zur Anwendung des Speichel absorbierenden Materials auf die Mundhöhle und Anweisungen für ein Behandlungsverfahren auf der Grundlage der nachgewiesenen Menge an Indikatorsubstanz für polymorphkernige Neutrophile enthält.

14. Verwendung nach Anspruch 13, wobei die Anweisungen zur Anwendung des Speichel absorbierenden Materials auf die Mundhöhle umfassen:
(a) Gewinnen einer Testprobe von Material aus dem Mund des Subjekts,
(b) Bestimmen der Menge an Indikatorsubstanz für polymorphkernige Neutrophile in der Testprobe,
(c) Vergleichen der Menge an Indikatorsubstanz für polymorphkernige Neutrophile in der Testprobe mit einer Kontrollprobe.

15. Verwendung nach Anspruch 13 oder 14, wobei das antimikrobielle Mittel ausgewählt ist aus der Gruppe bestehend aus halogeniertem Diphenylether, Triclosan, Kräuterextrakten oder ätherischen Ölen, Rosmarinextrakt, Thymol, Menthol, Eukalyptol, Methylsalicylat, Bisguanid-Antiseptika, Chlorhexidin, Alexidin oder Octenidin, Phenol-Antiseptika, Hexetidin, Povidoniodin, Delmopinol, Salifluor, Metallionen und deren Salzen, Zinkchlorid, Zinklactat, Zinkcitrat, Zinn(II)-fluorid und Zinn(II)-chlorid, Sanguinarin, Propolis, oxygenierenden Mitteln, Wasserstoffperoxid, gepuffertem Natriumperoxyborat oder Peroxycarbonat, Cetylpyridiniumchlorid, Magnolienextrakt, Magnolol, Honokiol, Butylmagnolol, Propylhonokiol und Gemischen davon.

## Revendications

1. Procédé de surveillance de l'efficacité d'un traitement de l'inflammation buccale dans un sujet, comprenant (a) se procurer un échantillon de test de matière buccale provenant dudit sujet, (b) déterminer la quantité de neutrophiles polymorphonucléaires ou d'une substance indicatrice de neutrophiles polymorphonucléaires dans ledit échantillon de test, (c) comparer la quantité de neutrophiles polymorphonucléaires ou de la substance indicatrice de neutrophiles polymorphonucléaires dans l'échantillon de test par rapport à un échantillon témoin, l'échantillon témoin étant un échantillon d'une matière buccale provenant du même sujet à un moment antérieur, et le sujet ayant été traité par un ou plusieurs agents antimicrobiens.

2. Procédé selon la revendication 1, dans lequel la matière buccale est la salive.

3. Procédé selon l'une des revendications 1 ou 2, dans lequel un taux augmenté de substance indicatrice de neutrophiles polymorphonucléaires dans l'échantillon de test indique un traitement inefficace de l'inflammation orale, et un taux diminué de substance indicatrice de neutrophiles polymorphonucléaires dans l'échantillon de test indique un traitement efficace de l'inflammation buccale.

4. Procédé selon l'une quelconque des revendications 1 à 3, dans lequel l'inflammation buccale est la gingivite, la périodontite ou l'halitose.

5. Procédé selon l'une quelconque des revendications 1 à 4, dans lequel l'échantillon de test est obtenu avec un tampon de prélèvement.

6. Procédé selon l'une quelconque des revendications 1 à 4, dans lequel l'échantillon de test est obtenu par expectoration.

7. Procédé selon l'une quelconque des revendications 1 à 6, dans lequel la substance indicatrice de neutrophiles polymorphonucléaires est la lactoferrine.

8. Procédé selon l'une quelconque des revendications 1 à 6, dans lequel la substance indicatrice de neutrophiles polymorphonucléaires est une leucocyte estérase.

9. Procédé selon l'une quelconque des revendications 1 à 6, dans lequel la substance indicatrice de neutrophiles polymorphonucléaires est la calprotectine.

10. Procédé selon l'une quelconque des revendications 1 à 9, dans lequel la matière buccale est mise en contact avec une matière absorbante contenant des réactifs pour déterminer la présence ou la quantité de substance indicatrice de neutrophiles polymorphonucléaires.

11. Procédé selon la revendication 10, dans lequel la matière absorbante est une bande ou dans une bandelette réactive.

12. Procédé selon l'une quelconque des revendications 1 à 11, dans lequel l'agent antimicrobien est choisi dans le groupe consistant en un diphényl éther halogéné, le triclosan, les extraits végétaux ou les huiles essentielles, l'extrait de romarin, le thymol, le menthol, l'eucalyptol, le salicylate de méthyle, les antiseptiques bisguanides, la chlorhexidine, l'alexidine ou l'octénidine, les antiseptiques phénoliques, l'hexétidine, la povidone iodée, le delmopinol, le salifluor, les ions métalliques et leurs sels, le chlorure de zinc, le lactate de zinc, le citrate de zinc, le fluorure stanneux et le chlorure stanneux, la sanguinarine, le propolis, les agents oxygénants, le peroxyde d'hydrogène, le peroxyborate ou le peroxycarbonate de sodium tamponné, le chlorure de cétyl pyridinium, l'extrait de magnolia, le magnolol, l'honokiol, le butyl magnolol, le propyl honokiol et leurs mélanges.

13. Utilisation d'un kit de diagnostic pour surveiller l'efficacité d'un traitement de l'inflammation buccale dans un sujet, dans laquelle l'utilisation comprend (a) se procurer un échantillon de test de matière buccale provenant dudit sujet, (b) déterminer la quantité de neutrophiles polymorphonucléaires ou de substance indicatrice de neutrophiles polymorphonucléaires dans ledit échantillon de test, (c) comparer la quantité de neutrophiles polymorphonucléaires ou de substance indicatrice de neutrophiles polymorphonucléaires dans l'échantillon de test à un échantillon de témoin, l'échantillon témoin étant un échantillon d'une matière buccale provenant du même sujet à un moment antérieur, le sujet ayant été traité par un ou plusieurs agents antimicrobiens, et le kit de diagnostic comprenant une matière absorbant la salive qui contient des réactifs pour déterminer la présence et/ou la quantité de substance indicatrice de neutrophiles polymorphonucléaires conjointement avec des instructions pour l'application de la matière absorbant la salive dans la cavité buccale et des instructions pour une méthode de traitement basée sur la quantité de substance indicatrice de neutrophiles polymorphonucléaires qui a été détectée.

14. Utilisation selon la revendication 13, dans laquelle les instructions pour l'application de la matière absorbant la salive à la cavité buccale comprennent :
(a) obtenir un échantillon de test de matière buccale à partir dudit sujet;
(b) déterminer la quantité de substance indicatrice de neutrophiles polymorphonucléaires dans ledit échantillon de test ;
(c) comparer la quantité de substance indicatrice de neutrophiles polymorphonucléaires dans l'échantillon de test à un échantillon témoin.

15. Utilisation selon l'une des revendications 13 ou 14, dans laquelle l'agent antimicrobien est choisi dans le groupe consistant en un diphényl éther halogéné, le triclosan, les extraits végétaux ou huiles essentielles, l'extrait de romarin, le thymol, le menthol, l'eucalyptol, le salicylate de méthyle, les antiseptiques bisguanides, la chlorhexidine, l'alexidine ou l'octénidine, les antiseptiques phénoliques, l'hexétidine, la povidone iodée, le delmopinol, le salifluor, les ions métalliques et leurs sels, le chlorure de zinc, le lactate de zinc, le citrate de zinc, le fluorure stanneux et le chlorure stanneux, la sanguinarine, le propolis, les agents oxygénants, le peroxyde d'hydrogène, le peroxyborate ou le peroxycarbonate de sodium tamponné, le chlorure de cétyl pyridinium, l'extrait de magnolia, le magnolol, l'honokiol, le butyl magnolol, le propyl honokiol et leurs mélanges.
